## Europäisches Patentamt
## European Patent Office
## Office européen des brevets

(11) Veröffentlichungsnummer: **0 224 157**
**B1**

(12) **EUROPÄISCHE PATENTSCHRIFT**

(45) Veröffentlichungstag der Patentschrift:
24.01.90

(51) Int. Cl.⁴: **A23L 1/221**, A23L 1/22,
A61K 9/52, A61K 9/48

(21) Anmeldenummer: 86115892.1

(22) Anmeldetag: 15.11.86

(54) Orale Zubereitungen von Knoblauch und Verfahren zu ihrer Herstellung.

(30) Priorität: 22.11.85 DE 3541304

(43) Veröffentlichungstag der Anmeldung:
03.06.87 Patentblatt 87/23

(45) Bekanntmachung des Hinweises auf die Patenterteilung:
24.01.90 Patentblatt 90/4

(84) Benannte Vertragsstaaten:
AT BE CH DE ES FR GB GR IT LI LU NL SE

(56) Entgegenhaltungen:
EP-A- 0 180 672
WO-A-83/03061
DE-A- 2 037 947
DE-A- 2 639 130

(73) Patentinhaber: R.P. Scherer GmbH, Gammelsbacher
Strasse 2 Postfach 1243, D-6930 Eberbach/Baden(DE)

(72) Erfinder: Hess, Heinz, Ersheimer Strasse 41,
D-6932 Hirschhorn(DE)
Erfinder: Mehn, Siegfried, Hirschweg 12,
D-6930 Eberbach-Igelsbach(DE)
Erfinder: Schönmann, Holger, Dr. Dipl.-Chem.,
Gerhard-Hauptmann-Strasse 15, D-6930 Eberbach(DE)

(74) Vertreter: Werner, Hans-Karsten, Dr. et al,
Deichmannhaus am Hauptbahnhof, D-5000 Köln 1(DE)

## Beschreibung

· Gegenstand der vorliegenden Erfindung sind orale Zubereitungen von Knoblauch enthaltend sowohl Knoblauchpulver als auch das Enzym Allinase.

Die bisher bekannten oralen Zubereitungen von Knoblauch enthalten entweder nur Knoblauchpulver und haben sich als nahezu wirkungslos erwiesen, oder enthalten sowohl Knoblauchpulver als auch das Enzym Allinase. Knoblauchpulver ist entweder eine gemahlene Droge oder ein getrockneter Drogenauszug, in denen das natürlicherweise vorkommende Enzym Allinase zerstört wurde. Die Zerstörung erfolgt üblicherweise durch kurzzeitige Erhitzung auf Temperaturen über 70°C, bei denen die übrigen Bestandteile des Knoblauchpulvers praktisch unverändert bleiben. Weiterhin sind chemische Methoden zur Zerstörung des Enzyms Allinase bekannt.

Zubereitungen, die sowohl Knoblauchpulver als auch das Enzym Allinase enthalten, sind instabil und besitzen den bekannten unangenehmen Geruch des Knoblauchs. Weitere bekannte Zubereitungen enthalten nur noch schwankende Restmengen des Enzyms Allinase und schwanken daher bezüglich Stabilität, Wirksamkeit und Geruchsbelästigung. Es war somit zumindest bisher nicht möglich, in reproduzierbarer Weise orale Knoblauchzubereitungen herzustellen, die allen Anforderungen an ein derartiges Präparat gerecht werden.

Die Erfindung hat sich die Aufgabe gestellt, orale Zubereitungen von Knoblauch zu entwickeln, welche sowohl das Knoblauchpulver in wirksamer Form als auch das Enzym Allinase enthalten, reproduzierbar und leicht herstellbar sind, lagerfähig sind, keine Geruchsbelästigung zeigen und dennoch nach der Einnahme die volle Wirksamkeit des Knoblauchs aufweisen.

Diese Aufgabe kann erfindungsgemäß dadurch gelöst werden, daß die beiden Komponenten räumlich voneinander getrennt vorliegen, jedoch nach der Einnahme wiedervereinigt werden.

Diese Aufgabe kann beispielsweise gelöst werden, dadurch daß jede der beiden Komponenten für sich mikroverkapselt und beide Arten von Mikrokapseln miteinander vermischt vorliegen. Weiterhin ist es möglich, das Enzym in Form sprüherstarrter Pellets, deren Fettgrundlage einen Schmelzpunkt unter 40°C aufweist, und das Knoblauchpulver in Form von sprüherstarrten Fettpellets zu bringen und beide Arten von Pellets miteinander zu vermischen. Bei der Vermischung dieser beiden Arten von Pellets kann es zweckmäßig sein, geringe Mengen von Maisstärke zuzumischen, die die Klebrigkeit der Pellets herabsetzt. Dieses Gemisch kann dann in an sich bekannter Weise in Hartgelatinekapseln abgefüllt werden.

Eine weitere Lösungsmöglichkeit besteht darin, das Knoblauchpulver und das Enzym in tablettierbare Form zu bringen und jedes dieser beiden Vormischungen in an sich bekannter Weise in eine Schicht einer Zweischichttablette zu verpressen.

Schließlich ist es möglich, die beiden Komponenten in einer Mischung von gesättigten Triglyceriden und Pflanzenöl zu suspendieren und in dieser Form in an sich bekannter Weise in Weichgelatinekapseln abzufüllen.

Alle diese Zubereitungsformen haben sich als stabil erwiesen, gestatten jedoch den beiden Komponenten nach der Einnahme miteinander zu reagieren und die wirksamen Bestandteile des natürlichen Knoblauchs zu entwickeln.

Gegenstand der vorliegenden Erfindung sind somit die oben beschriebenen oralen Zubereitungsformen sowie die Verfahren zu ihrer Herstellung gemäß obiger Patentansprüche.

Weitere Einzelheiten, typische Ausführungsformen der oralen Zubereitungen und der Verfahren zu ihrer Herstellung sind in den nachfolgenden Beispielen näher erläutert.

### Beispiel 1

#### Fettpellets in Hartkapseln

20 kg Knoblauchpulver werden in 80 kg geschmolzenem Glycerinmonostearat feinst dispergiert. Die heiße Suspension wird durch eine rotierende Düse mit 0,5 mm Bohrung durch Sprüherstarrung pelletisiert. 5 kg Allinase (Pulver) werden in 20 kg geschmolzenem Hartfett, dessen Temperatur 40°C nicht übersteigen darf, ebenfalls durch Sprüherstarrung pelletisiert. Nach dem Absieben der geeigneten Kornfraktionen werden beide Pelletarten in einen Konusmischer zusammen mit 0,25% Maisstärke gemischt. Von der fertigen Pelletmischung werden 501,25 mg pro Hartkapsel abgefüllt.

### Beispiel 2

#### Zweischicht-Filmtablette

20 kg Knoblauchpulver werden in einer Schmelze aus 35,8 kg Cetylalkohol und 1,7 kg Lecithin suspendiert. Die erkaltete Suspension wird in geeigneter Weise grob pulverisiert. Dem Pulver werden 1,5 kg Maisstärke und 1 kg pyrogene Kieselsäure (Aerosil 200$^R$) zugesetzt (Pulver 1).

Aus 5 kg Allinase (Pulver) 13 kg gehärtetes Rizinusöl, 5 kg Maisstärke, 1 kg Stearinsäure und 1 kg Methylcellulose wird durch Trockenmischen ein Granulat hergestellt (Pulver 2).

Auf einer Zweischichttablettenpresse werden aus 240 mg Pulver 1 und 100 mg Pulver 2 Tablettenkerne hergestellt. Die Kerne werden mit einem feuchtigkeitsabweisenden Schellackfilm vorlackiert und danach mit einer Suspension aus Pharmacoat 606$^R$, Titandioxid, PEG 6000 mit Eisenoxidgelb in Wasser gecoated.

### Beispiel 3

#### Weichgelatinekapseln

20 kg Allinase (Pulver) und 80 kg Knoblauchpulver werden in einer Mischung aus 54 kg gesättigten Triglyceriden und 146 kg Pflanzenöl suspendiert. Die Suspension wird in geeigneter Weise homogenisiert, gesiebt und entlüftet. Jeweils 300 mg der Suspension werden in bekannter Weise in Weichgelatinekapseln abgefüllt.

**Patentansprüche**

1. Orale Zubereitungen von Knoblauch enthaltend sowohl Knoblauchpulver als auch das Enzym Allinase, dadurch gekennzeichnet, daß die beiden Komponenten räumlich voneinander getrennt vorliegen, jedoch nach der Einnahme wiedervereinigt werden.

2. Zubereitungen gemäß Anspruch 1, dadurch gekennzeichnet, daß jede der beiden Komponenten für sich mikroverkapselt vorliegt und beide Arten von Mikrokapseln miteinander vermischt vorliegen.

3. Zubereitungen gemäß Ansprüch 1, dadurch gekennzeichnet, daß das Enzym in Form sprüherstarrter Pellets, deren Fettgrundlage einen Schmelzpunkt unter 40°C aufweist, und das Knoblauchpulver in Form sprüherstarrter Fettpellets vorliegt und beide Arten von Pellets miteinander vermischt vorliegen.

4. Zubereitungen gemäß Anspruch 3, dadurch gekennzeichnet, daß das Gemisch in Hartgelatinekapseln abgefüllt ist.

5. Zubereitungen gemäß Anspruch 1, dadurch gekennzeichnet, daß das Knoblauchpulver und das Enzym in je einer Schicht einer Zweischichttablette vorliegen.

6. Zubereitungen gemäß Anspruch 1, dadurch gekennzeichnet, daß die beiden Komponenten in einer Mischung von gesättigten Triglyceriden und Pflanzenöl suspendiert vorliegen und in Weichgelatinekapseln abgefüllt sind.

7. Verfahren zur Herstellung von oralen Zubereitungen von Knoblauch enthaltend sowohl Knoblauchpulver als auch das Enzym Allinase, dadurch gekennzeichnet, daß jede der beiden Komponenten für sich mikroverkapselt wird und beide Arten von Mikrokapseln miteinander vermischt werden.

8. Verfahren zur Herstellung von oralen Zubereitungen von Knoblauch enthaltend sowohl Knoblauchpulver als auch das Enzym Allinase, dadurch gekennzeichnet, daß
a) das Enzym in einem geschmolzenen Hartfett bei Temperaturen bis 40°C dispergiert, und durch Sprüherstarrung pelletisiert wird,
b) das Knoblauchpulver in geschmolzener Fettgrundlage dispergiert und durch Sprüherstarrung pelletisiert wird,
c) beide Arten von Pellets gegebenenfalls unter Zumischung von Maisstärke miteinander vermischt und in Hartgelatinekapseln abgefüllt werden.

9. Verfahren zur Herstellung von oralen Zubereitungen von Knoblauch enthaltend sowohl Knoblauchpulver als auch das Enzym Allinase, dadurch gekennzeichnet, daß sowohl das Knoblauchpulver als auch das Enzym in tablettierbare Form gebracht und in einer Zweischichttablettenpresse zu Zweischichttabletten verarbeitet werden.

10. Verfahren zur Herstellung von oralen Zubereitungen von Knoblauch enthaltend sowohl Knoblauchpulver als auch das Enzym Allinase, dadurch gekennzeichnet, daß beide Komponenten in einer Mischung von gesättigten Triglyceriden und Pflanzenöl suspendiert und in in an sich bekannter Weise in Weichgelatinekapseln abgefüllt werden.

**Claims**

1. Oral preparations of garlic, containing garlic powder as well as the enzyme allinase, characterized in that both of the components are present spatially separated from one another, but will be recombined once taken in.

2. The preparations according to claim 1, characterized in that either of the two components is present microencapsulated by itself and both types of microcapsules are present in admixture with each other.

3. The preparations according to claim 1, characterized in that the enzyme is in the form of spray-solidified pellets, the fat base of which has a melting point below 40°C, and the garlic powder is in the form of spray-solidified fat pellets, and both types of pellets are present in admixture with each other.

4. The preparations according to claim 3, characterized in that the mixture has been filled into hard gelatin capsules.

5. The preparations according to claim 1, characterized in that the garlic powder and the enzyme each are present in one layer of a two-layer tablet.

6. The preparations according to claim 1, characterized in that both of the components are present suspended in a mixture of saturated triglycerides and vegetable oil and have been filled into soft gelatin capsules.

7. A process for preparing oral preparations of garlic containing garlic powder as well as the enzyme allinase, characterized in that either of the components are microencapsulated by itself and both types of microcapsules are mixed with each other.

8. A process for preparing oral preparations of garlic containing garlic powder as well as the enzyme allinase, characterized in that
a) the enzyme is dispersed in a molten hard fat at a temperature of below 40°C and the mixture is pelletized by spray solidifaction,
b) the garlic powder is dispersed in a molten fat base and the mixture is pelletized by spray solidifaction,
c) both kinds of pellets are mixed with each other, optionally with the addition of corn starch, and are filled into hard gelatin capsules.

9. A process for preparing oral preparations of garlic containing garlic powder as well as the enzyme allinase, characterized in that the garlic powder as well as the enzyme are brought in a tablet form and on a two-layer tablet press are processed to form two-layer tablets.

10. A process for preparing oral preparations of garlic containing garlic powder as well as the enzyme allinase, characterized in that the garlic powder as well as the enzyme are suspended in a mixture of saturated triglycerides and vegetable oil and in a per se known manner are filled into soft gelatin capsules.

**Revendications**

1. Préparations orales d'ail contenant tant de la poudre d'ail que l'enzyme allinase, caractérisées en ce que les deux composants sont disposés séparés l'un de l'autre dans l'espace mais sont pourtant réunis à nouveau après ingestion.

2. Préparations selon la revendication 1, caractérisées en ce que chacun des deux composants est présent séparément sous forme de microcapsules, et en ce que les deux types de microcapsules sont mélangés l'un à l'autre.

3. Préparations selon la revendication 1, caractérisées en ce que l'enzyme est présente sous forme de boulettes, ou pellets, solidifiées par pulvérisation, dont la base grasse présente un point de fusion inférieur à 40°C, et la poudre d'ail sous forme de boulettes, ou pellets, grasses, solidifiées par pulvérisation, et en ce que les deux types de boulettes sont mélangés l'un à l'autre.

4. Préparations selon la revendication 3, caractérisées en ce que le mélange est placé dans des capsules en gélatine dure.

5. Préparations selon la revendication 1, caractérisées en ce que la poudre d'ail et l'enzyme se trouvent chacun dans une couche d'un comprimé à deux couches.

6. Préparations selon la revendication 1, caractérisées en ce que les deux composants sont présents en suspension dans un mélange de triglycérides saturés et d'huile végétale, et sont versés dans des capsules en gélatine molle.

7. Procédé de fabrication de préparations orales d'ail contenant à la fois de la poudre d'ail et l'enzyme allinase, caractérisé en ce que chacun des deux composants est emballé séparément en microcapsules et en ce que les deux types de microcapsules sont mélangés l'un à l'autre.

8. Procédé de fabrication de préparations orales d'ail contenant à la fois de la poudre d'ail et l'enzyme allinase, caractérisé en ce que

a) l'enzyme est dispersé à des températures allant jusqu'à 40°C dans une graisse dure fondue, et qu'il est transformé en boulettes, ou pellets, par solidification par pulvérisation,

b) la poudre d'ail est dispersée dans une base grasse fondue et transformée en boulettes, ou pellets, par solidification par pulvérisation,

c) les deux types de boulettes, ou pellets, sont mélangés, avec addition éventuelle d'amidon de maïs et sont versés dans des capsules en gélatine dure.

9. Procédé de fabrication de préparations orales d'ail contenant à la fois de la poudre d'ail et l'enzyme allinase, caractérisé en ce que tant la poudre d'ail que l'enzyme sont amenés à des formes susceptibles de constituer des comprimés et mis sous forme des comprimés à deux couches dans une presse à comprimés en deux couches.

10. Procédé de fabrication de préparations orales d'ail contenant tant de la poudre d'ail que l'enzyme allinase, caractérisé en ce que les deux composants sont mis en suspension dans un mélange de triglycérides saturés et d'huile végétale et incorporés d'une manière connue en soi à des capsules en gélatine molle.